# EUROPEAN PATENT SPECIFICATION

(11) **EP 3 452 581 B1**
(45) Date of publication and mention of the grant of the patent: **11.01.2023**
(21) Application number: 17792305.9
(22) Date of filing: 03.05.2017
(51) Int. Cl.: C12N 5/0789, C12N 5/02, C12N 5/078

(54) **4HPR AND ITS USE IN THE CULTURING OF HEMATOPOIETIC STEM CELLS**
4HPR UND DESSEN VERWENDUNG IN DER ZÜCHTUNG VON HÄMATOPOETISCHEN STAMMZELLEN
4HPR ET SON UTILISATION DANS LA CULTURE DE CELLULES SOUCHES HÉMATOPOÏÉTIQUES

(30) Priority: 03.05.2016 US 201662331019 P
(43) Date of publication of application: 13.03.2019
(73) Proprietor: University Health Network, Toronto, Ontario M5G 2C4 (CA)
(72) Inventor: DICK, John, Toronto, Ontario M4E 2ZS (CA); XIE, Stephanie, Toronto, Ontario M5G 2C4 (CA); LAURENTI, Elisa, Mill Road Cambridge CB1 3UG (GB); PRAT, Laura Garcia, Toronto, Ontario M5G 2A1 (CA); FERRARI, Robin Thomas, 75014 Paris (FR)
(74) Representative: AWA Sweden AB
(86) International application number: PCT/CA2017/000107
(87) International publication number: WO 2017/190214

(56) References cited:
- WO-A2-03/062369
- WO-A2-2007/033331
- CN-A- 101 623 277
- US-A1- 2010 008 896
- US-B2- 8 088 822
- ZHANG HUI ET AL: "Preferential eradication of acute myelogenous leukemia stem cells by fenretinide", PROCEEDINGS OF THE NATIONAL ACADEMY OF SCIENCES OF THE UNITED STATES OF AMERICA, vol. 110, no. 14, 2 April 2013 (2013-04-02), pages 5606-5611, XP002755484, DOI: 10.1073/PNAS.1302352110
- J. P. CHUTE: "Inhibition of aldehyde dehydrogenase and retinoid signaling induces the expansion of human hematopoietic stem cells", PROCEEDINGS OF THE NATIONAL ACADEMY OF SCIENCES, vol. 103, no. 31, 1 August 2006 (2006-08-01), pages 11707-11712, XP055056573, ISSN: 0027-8424, DOI: 10.1073/pnas.0603806103
- ANTHONY E. BOITANO ET AL: "Aryl Hydrocarbon Receptor Antagonists Promote the Expansion of Human Hematopoietic Stem Cells", SCIENCE, vol. 329, no. 5997, 5 August 2010 (2010-08-05), pages 1345-1348, XP055558705, ISSN: 0036-8075, DOI: 10.1126/science.1191536
- IMAN FARES ET AL: "Cord blood expansion. Pyrimidoindole derivatives are agonists of human hematopoietic stem cell self-renewal", SCIENCE (NEW YORK, N.Y.), vol. 345, no. 6203, 19 September 2014 (2014-09-19), pages 1509-1512, XP055370388, United States
- XIE STEPHANIE ZHI-JUAN ET AL: "Sphingolipid Perturbation Activates Proteostasis Programs to Govern Human Hematopoietic Stem Cell Self-Renewal", BLOOD, vol. 132, no. Suppl. 1, 29 November 2018 (2018-11-29), page 170, XP002795058, PHILADELPHIA,PA,US ISSN: 0006-4971, DOI: 10.1182/blood-2018-170
- PARK, B. ET AL.: 'Hematopoietic stem cell expansion and generation: the ways to make a breakthrough' BLOOD RESEARCH, [Online] vol. 50, no. 4, December 2015, ISSN 2288-0011 pages 194 - 203, XP055435854 ISSN: 2288-0011 Retrieved from the Internet: <URL:http://dx.doi.Org/10.5045/br.2015.50.4 .194> [retrieved on 2017-08-14]
- HO T. T. ET AL.: 'Autophagy maintains the metabolism andfunction of young and old stem cells' NATURE, [Online] vol. 543, 09 March 2017, ISSN 1476-4687 pages 205 - 210, XP055435861 ISSN: 1476-4687 Retrieved from the Internet: <URL:http://palgrave. nature .com/ nature /journal/v543/n7644/full/ nature 21388.html>

## Description

### REFERENCE TO RELATED APPLICATION

This application claims priority to U.S. Provisional Patent Application No. 62/331,019 filed on May 3, 2016.

### FIELD OF THE INVENTION

The invention relates to culturing/expanding hematopoietic stem cells and more particularly to the use of N-(4hydroxyphenyl)retinamide (4HPR) for improving or preserving hematopoietic stem cell renewability.

### BACKGROUND OF THE INVENTION

Limited numbers of hematopoietic stem and progenitor cells (HSPC) are barriers to the efficacy and prominent usage of human umblical cord blood (UCB) units for recovery from bone marrow transplantation (BMT). UCB are an under-utilized source for BMT, but may serve as the only allogenic source available for a vast majority of the non-Causasian population. For more than two decades, different strategies have been employed to expand unfractionated or stem cell enriched human CB CD34+/CD133+ cells in culture. Early approaches utilized combinations of cytokines that were being discovered at the time, alone or with stromal co-cultures. While massive expansion of CD34+ cells was achieved, assessment with xenograft repopulation assays revealed concomitant loss of HSC; further substantiated in clinical trial findings with no contribution to patient repopulation. Thus, a strategy to pursue ex vivo expansion of immunophenotypic CD34+ cells is not by itself sufficient for yielding functionally expanded LT-HSC for HSCT. The most advanced methods that are currently in trial today for ex vivo expansion have evolved from screening of compound libraries (SR1 and UM171) with impressive expansion of CD34+; encouraging clinical data are just now emerging (Boitano, et al, Science, 2010; Fares, et al, Science, 2014; Wagner, et al, Cell Stem Cell, 2016). Better culture systems, cytokine conditions, delivery methods and prominently small molecule agonists for HSPC expansion in vitro have been found, but whether these expansion methods directly act on the HSC and increases long-term HSC self-renewal is limited.

HSC are the rare population of cells that maintain life-long blood production. HSC have distinct responses to stress stimuli including metabolic stress, DNA damage, and endoplastic reticulum (ER) stress from the rest of the hematopoietic hierarchy. Targeting such pathways are able to alter the functional output of the HSC in both self-renewal and multipotency. HSC are predisposed to choose apoptosis if stress stimuli are not resolved to maintain HSC pool integrity and avoid malignancy. However, HSC function can also be enhanced by selectively activating stress pathway components such as the co-chaperone ERDJ4 to enhance ER protein folding (van Galen, Nature, 2014). Macroautophagy, hereafter referred to as autophagy, is a stress response process critical for protecting mouse HSC from metabolic stress and aging (Warr, et al, Nature 2013; Ho, et al, Nature 2017). Specifically, only mouse HSC and not committed progenitors hold the capacity to activate protective autophagy upon stress stimuli (Warr, Nature, 2013). Thus, HSC contain characteristics that have potential for selective activation for clinical applications such as HSCT.

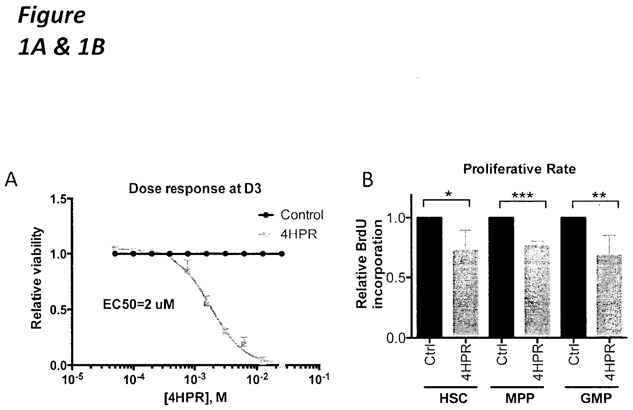

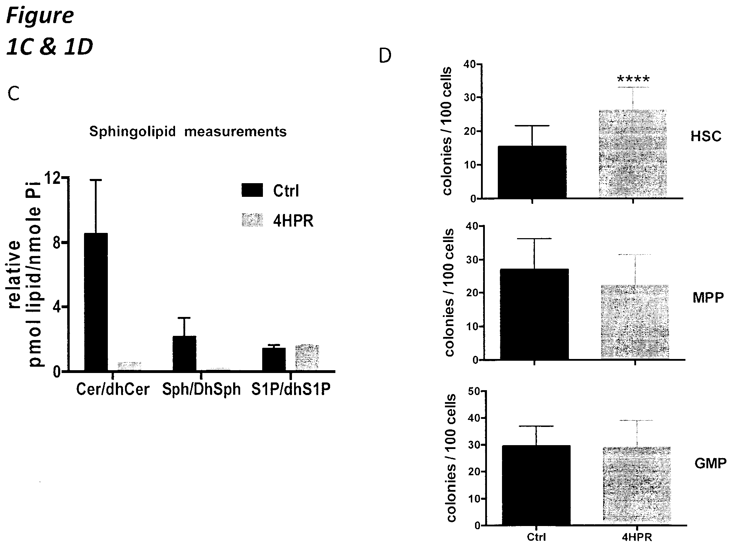

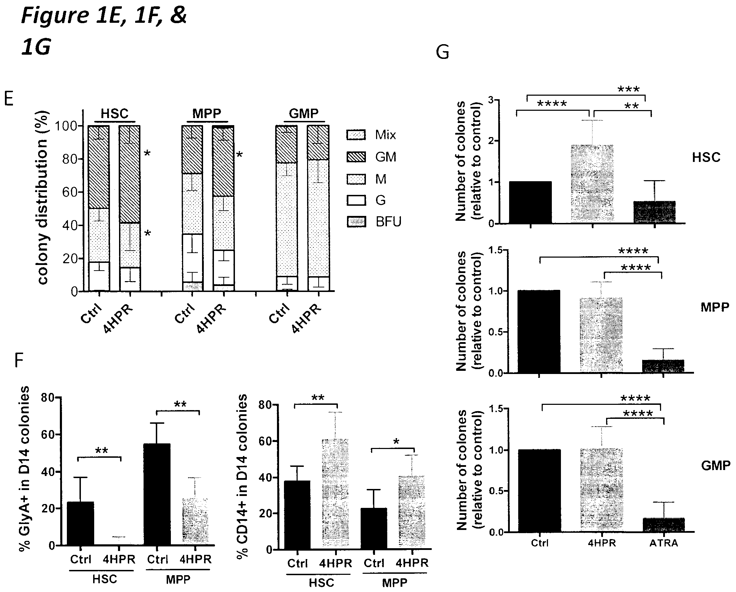

Research paper Anthony E. Boitano et al: " Aryl Hydrocarbon Receptor Antagonists Promote the Expansion of Human Hematopoietic Stem Cells", SCIENCE, vol . 329, no. 5997, 5 August 2010 (2010-08-05), pages 1345-1348, shows that StemRegenin 1 promotes the ex vivo expansion of C034+ cells by antagonizing the aryl hydrocarbon receptor, thus disclosing methods wherein the ex vivo expansion of HSCs is stimulated by antagonizing the aryl hydrocarbon receptor. Research paper Iman Fares et al: "Cord blood expansion. Pyrimidoindole derivatives are agonists of human hematopoietic stem cell self-renewal, SCIENCE (NEW YORK, N.Y.), vol. 345, no. 6203, 19 September 2014 (2014-09-19), pages 1509-1512, discloses that pyrimidoindole derivatives, such as UM171, stimulate the ex vivo expansion of human cord blood hematopoietic stem cells (figures 1-3), thus disclosing methods wherein the ex vivo expansion of HSCs is stimulated by using pyrimidoindole derivatives in the culture medium.

### SUMMARY OF THE INVENTION

The invention is set out in the appended set of claims.

In an aspect, there is provided a use of N-(4hydroxyphenyl)retinamide (4HPR) for improving or preserving hematopoietic stem cell (HSC) renewability during *ex-vivo* expansion of a population of cells comprising HSCs in an expansion medium.

### BRIEF DESCRIPTION OF THE FIGURES

These and other features of the preferred embodiments of the invention will become more apparent in the following detailed description in which reference is made to the appended drawings wherein:
**Figure** 1 shows pleiotrophic DEGS1 inhibitor fenretinide/4HPR selectively increases cloning efficiency in human HSC. (A) Dose response of varying [4HPR] in CB at day 3 post-treatment showing 2uM 4HPR gives the relative half maximal number of viable cells compared to control. All experiments following are at this 4HPR concentration unless otherwise indicated. (B) Relative % BrdU incorporation following 4 or 8 hours labeling at 3 days post-treatment with the indicated sorted cell populations shows 4HPR decreases the proliferation rate of primitive CB subpopulations in culture (n=4). (C) LC/MS was used to profile the ceramide (Cer), dihydroceramide (dhCer), sphingosine (sph), dihydrosphingosine (dhSph), S1P and dihydro-S1P (dhS1P) levels in lin- CB cells cultured for 8 days with control or 4HPR (n=2). The relative levels of lipid/dihydro-lipid show that 4HPR causes accumulation of dhCer and decreases total ceramide. (D) HSC, MPP, or GMP were sorted into methylcellulose for CFC assays in the presence of control or 4HPR and colonies were blindly scored following 10-11 days for (D) colonies/100 cells and ((E) colony distribution are shown (n=7). (F) Flow cytometry for erythroid (GlyA+) and monocytic (CD14+) markers in live cells from pooled CFC colonies were scored (n=6). (F) Relative number of colonies from CFC assays comparing the effect of 4HPR or ATRA (n=3) on sorted HSC, MPP or GMP.

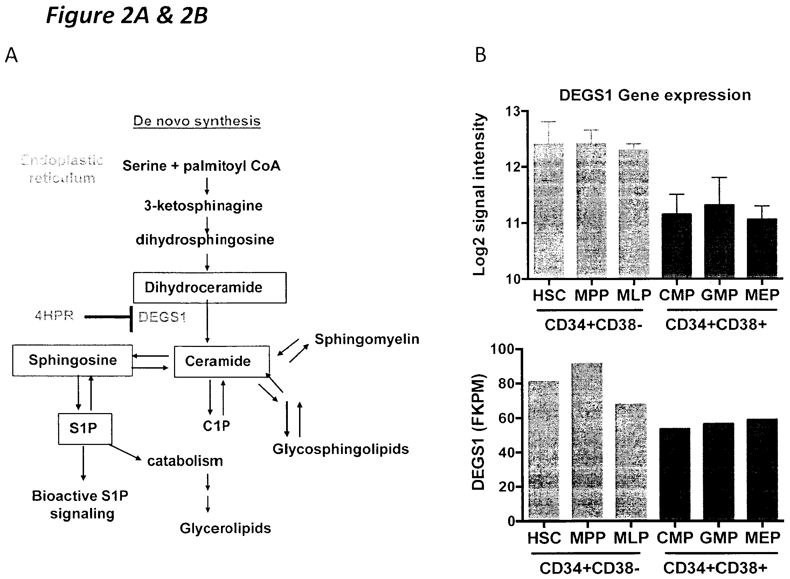

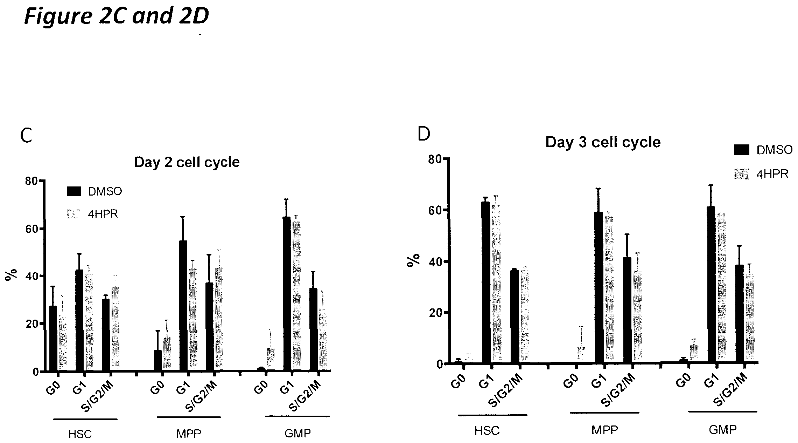

**Figure** 2 shows (A) Sphingolipid metabolism schematic showing DEGS1 as a biosynthetic enzyme in the de novo sphingolipid synthesis, which occurs in the endoplastic reticulum. 4HPR is a small molecule that can inhibit the activity of DEGS1. (B) DEGS1 gene expression for the CB hierarchy is show from illumina microarray analysis (Laurenti, et al, 2013) and RNA-sequencing (Zandi et al.) Cell cycle analysis for Ki67 and DNA content by flow cytometry of sorted HSC, MPP or GMP at (C) Day 2 (n=3) or (D) Day 3 post treatment (n=2) with vehicle control or4HPR shows 4HPR does not significantly prevent cells from transiting through the cell cycle.

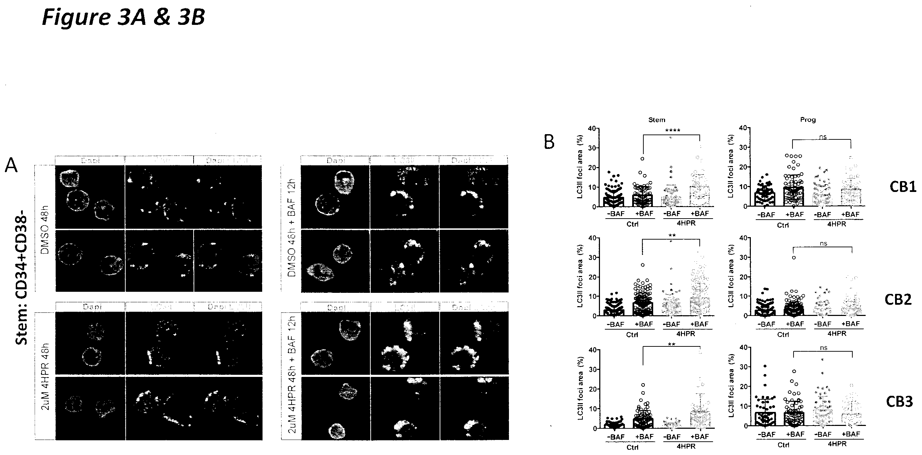

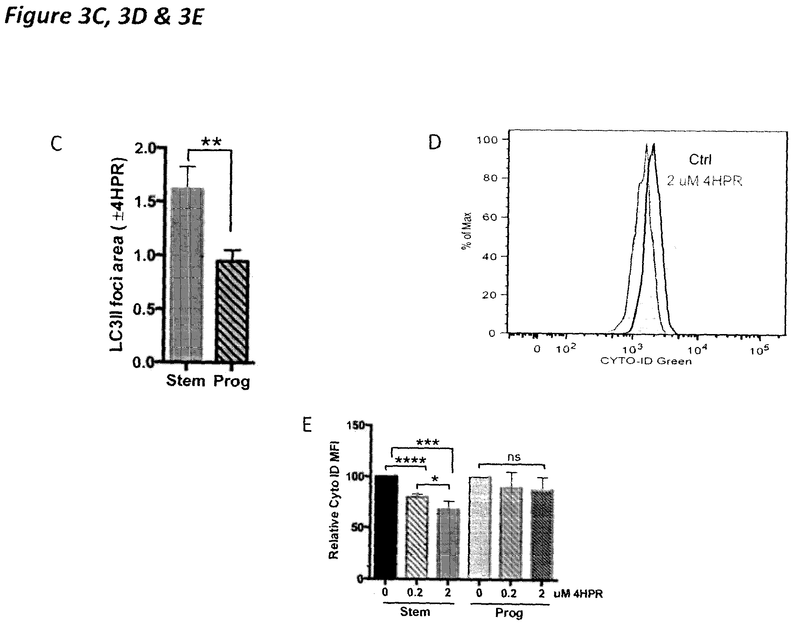

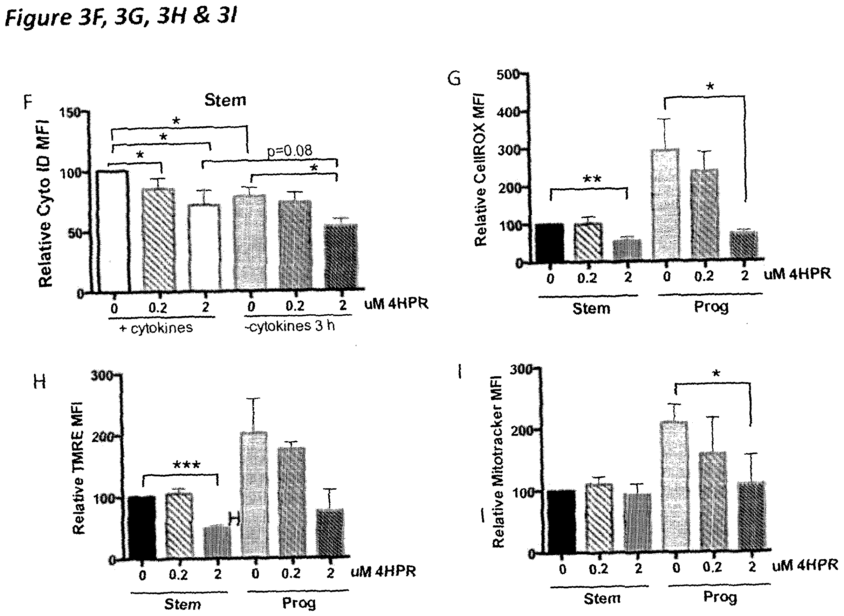

**Figure** 3 shows 4HPR activates autophagic flux and decreases ROS and mitochondrial membrane potential in HSPC during ex vivo culture. (A)Representative immunofluorescence images taken by confocal microscopy for DAPI (blue) and LC3II staining (green) for CD34+CD38- cells (stem) following 2 days of treatment with DMSO control or 2uM 4HPR with and without the autophagy inhibitor baflomycin A1 (BAF) to assay for basal autophagy and autophagic flux. (B) Quantitation of LC3II foci area as a percentage of total cell area for stem and prog (CD34+38+) cells from three independent CB using R and Image J software. (C) LCII foci area is increased with 4HPR treatment relative to control in the presence of BAF only in stem populations and not Prog populations. (D) Representative flow cytometry histogram plot of cyto-ID fluorescence intensity of control (black) and 4HPR (green) treated stem cells to assay for autophagic flux. Lower mean fluorescence intensity (MFI) indicates more turnover of autophagosomes and thus increased autophagic flux. (E) Relative cyto-ID flux for 0 (vehicle control), 0.2 uM and 2 uM 4HPR in stem and prog cell populations at 2 days post-treatment (n=4), shows increasing concentrations of 4HPR induces autophagic flux only in stem, not progenitor cells. (F) Cytokine withdrawal for 3 hours activates autophagic flux in CB stem cells to similar levels as 2 uM 4HPR as assayed by cyto-ID flow cytometry at day 2 post-treatment (n=3). Flow cytometry analysis of stem or prog cells following 2 days of treatment with indicated concentrations of 4HPR for (G) ROS with CELLROX, (H) mitochondrial membrane potential with TMRE and (I) mitochondrial mass with mitotracker green shows 4HPR decreases ROS species and mitochondrial membrane activity without significantly altering mitochondrial mass in stem cells. 4HPR treatment in progenitor cells does decrease mitochondrial mass.

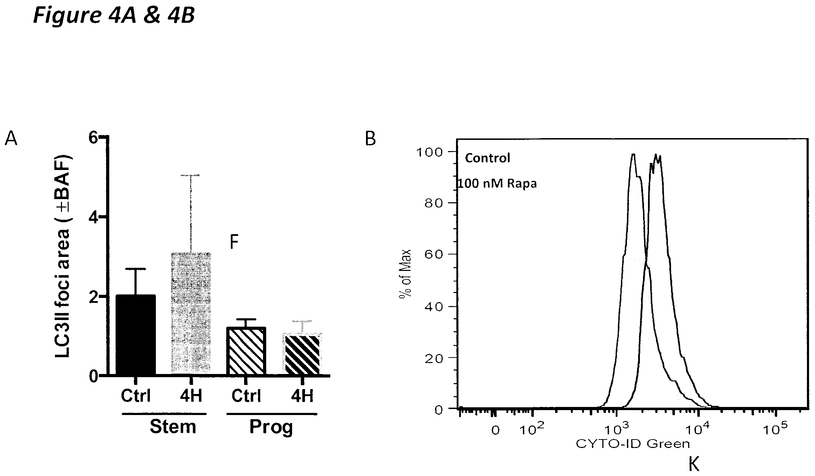

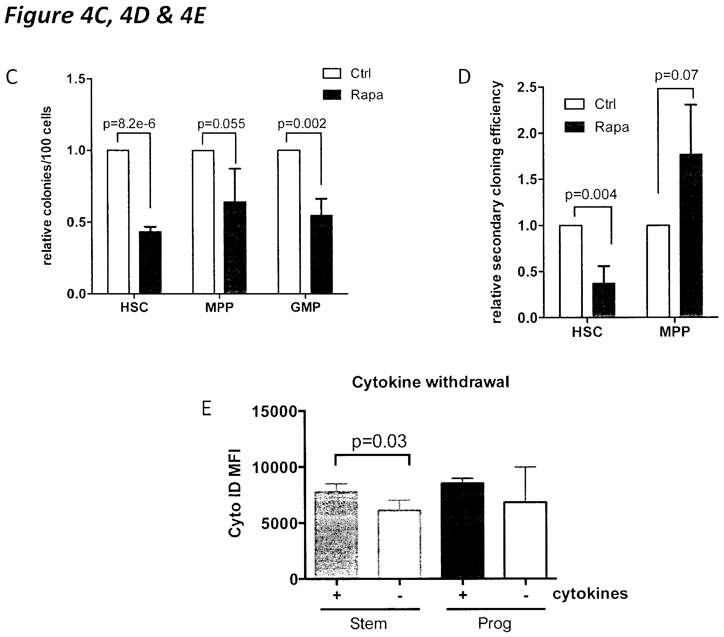

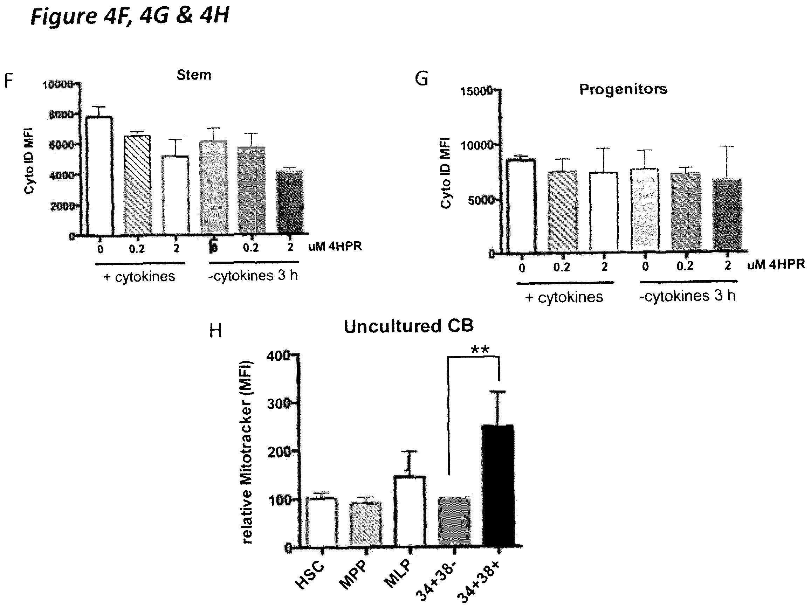

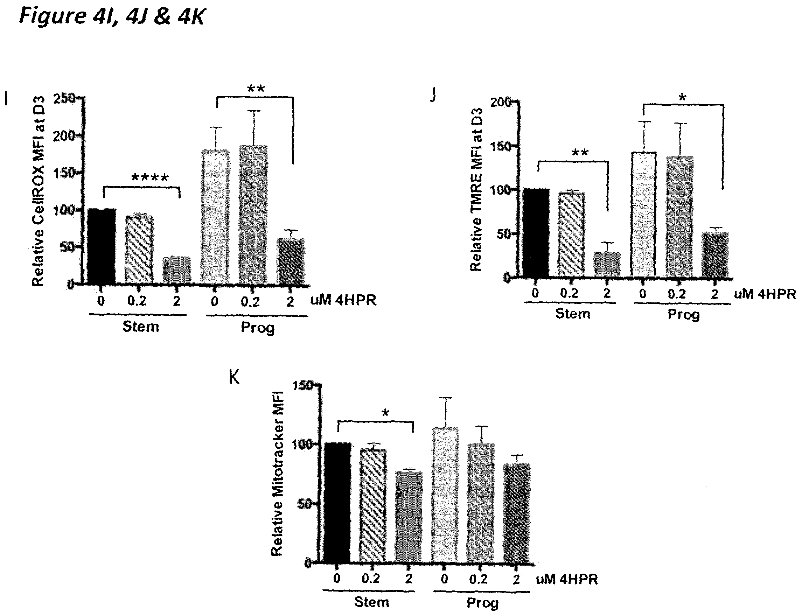

**Figure** 4 shows (A) Autophagic flux quantitation as a ratio of LC3II foci area with and without BAF for Figure 3B (n=3). (B). Representative cyto-ID flow cytometry plot of CB progenitor cells treated with 100 nM rapamycin to activate autophagy via mTOR inhibition as positive control for Figure 3E and F. (C) The relative number of colonies scored following DMSO control (ctrl) or 100 nM Rapamycin treatment of sorted HSC, MPP, and GMP in CFC assays at day 11 (n=3) shows rapamycin decreases colony output from CB HSPC. (D) The relative secondary cloning efficiency resulting from serial replating of HSC or MPP groups from C shows that initial rapamycin treatment increases progenitor function in this in vitro assay for MPP, but not HSC. (E) Cyto ID MFI following 3 hour cytokine withdrawal at 2 days post in vitro culture shows only stem, but not prog cells activate autophagic flux upon cytokine withdrawal. (F) CytolD MFI measurements for Figure 3F and the comparable data for (G) progenitor samples to illustrate autophagic flux is not significantly induced in progenitor cells treated with 4HPR even upon cytokine withdrawal. (H) Relative mitochondrial mass of indicated CB hierarchy populations from fresh, uncultured cord blood as assayed by mitotracker green flow cytometry shows CD34+CD38+ progenitor cells have 2-fold more mitochondrial mass than CD34+CD38-stem cells. This difference in mitochondrial content is lost by day 3 post culture (See Fig 3K). Flow cytometry analysis at D3 post-treatment with indicated concentrations of 4HPR in stem or progenitor CB cells for (I) ROS, (J) mitochondrial membrane potential and (K) mitochondrial mass indicates that 4HPR decreases mitochondrial mass by day 3 post-treatment in stem cells.

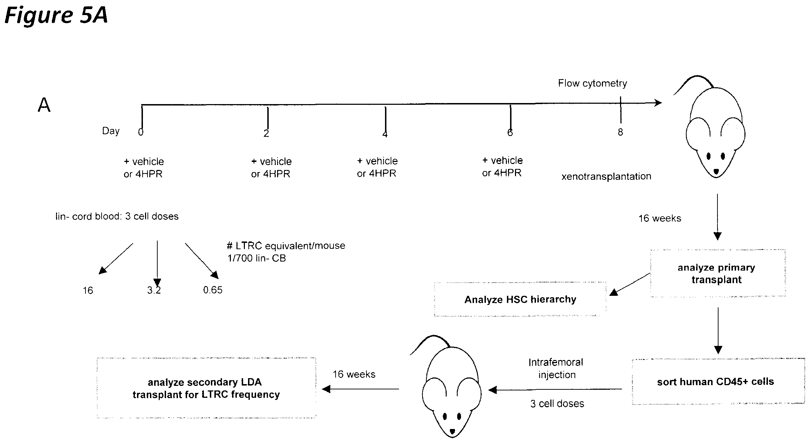

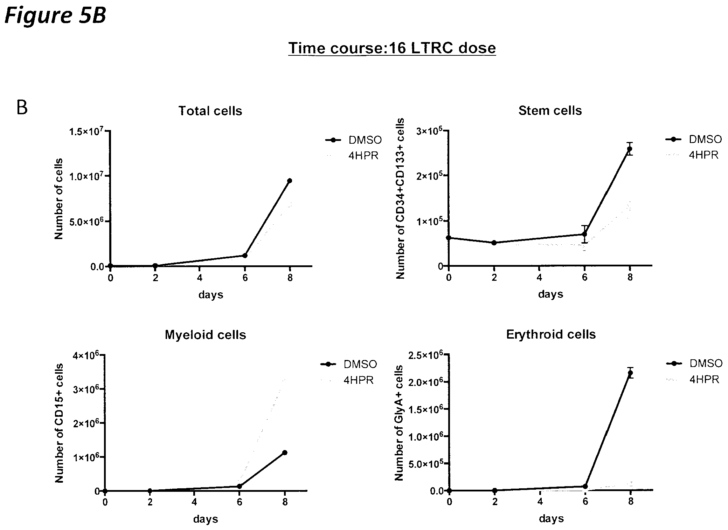

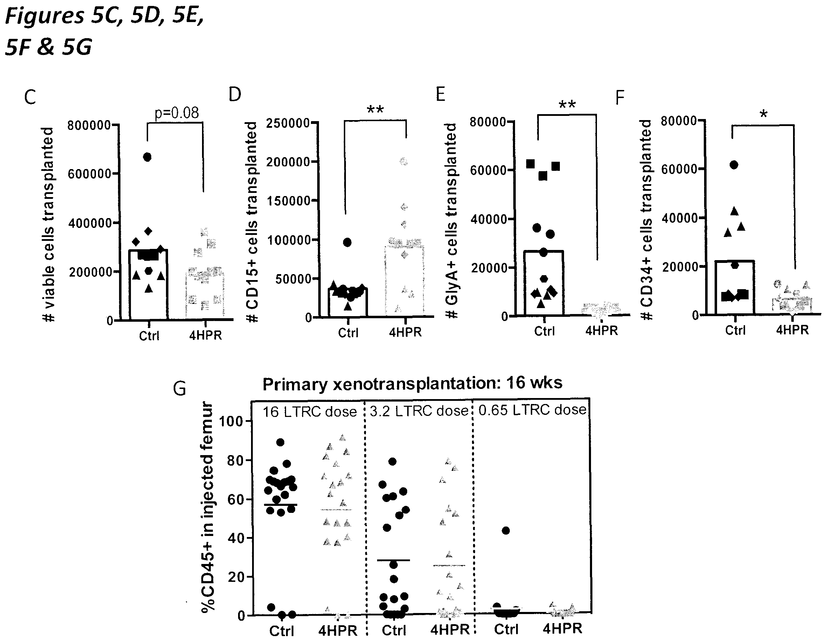

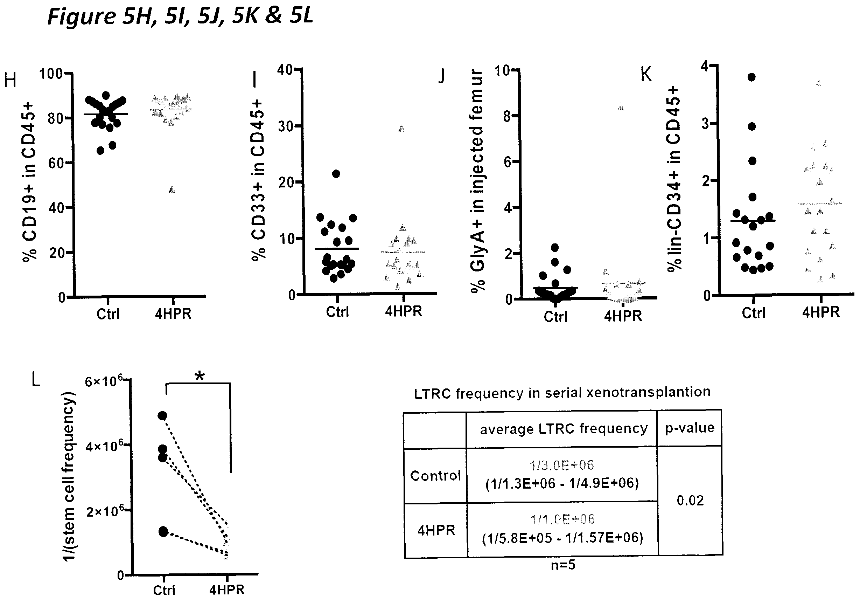

Figure 5 shows ex vivo expansion of human cord blood with 4HPR preserves HSC self-renewal following xenotransplantation. In vitro treatment of primitive cord blood with 4HPR preserves HSC function in xenotransplantation. (A) Experimental scheme (B) Representative FACS analysis data of in vitro culture for 16 HSC dose time course (n=3). The number of (C) viable cells, (D) CD15+ myeloid cells, (E) GlyA+ erythroid cells, and (F) CD34+ primitive cells measured for the16 LTRC dose with HTS flow cytometry analysis at day 8 post-treatment for vehicle control or 4HPR prior to xenotransplantation (n=5 biological replicates, 4 in technical triplicate, marked with different symbols). (G) Human CD45+ engraftment at 16 weeks post transplant in injected femur (n=4, 5 mice/biological experiment). Lineage analysis of control or 4HPR transplanted mice from figure 5G for (H) B lymphoid, (I) myeloid, (J) erythroid, and (K) primitive CD34+ at the 16 LTRC dose. (L) CD45+ cells were isolated from 5 biological experiments of the 16 LTRC dose primary mice and transplanted at limiting doses into secondary recipients for 16 weeks. Human CD45+ marking of >0.1% was considered positive for secondary engraftment. Limiting dilutation analysis was performed using ELDA software and the long-term repopulating cell (LTRC) frequency for each biological replicate plotted and the average LTRC frequency calculated.

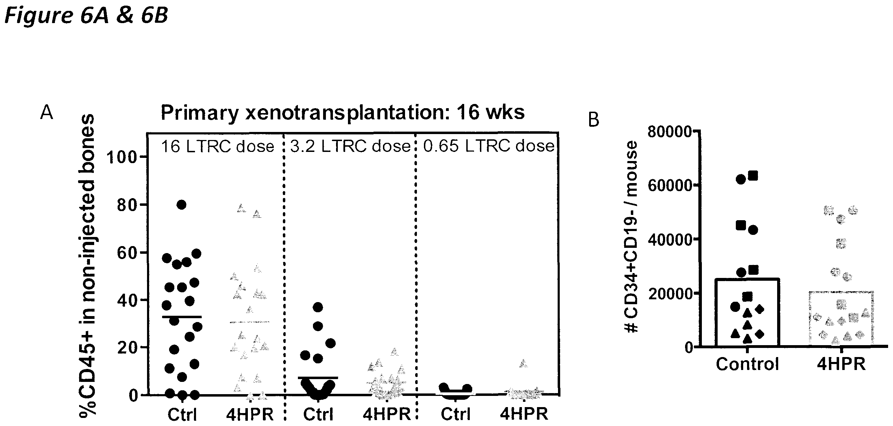

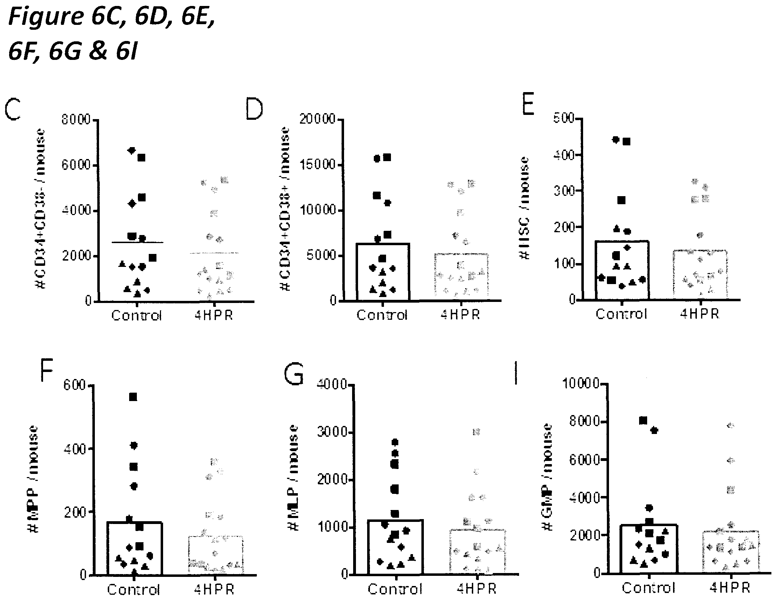

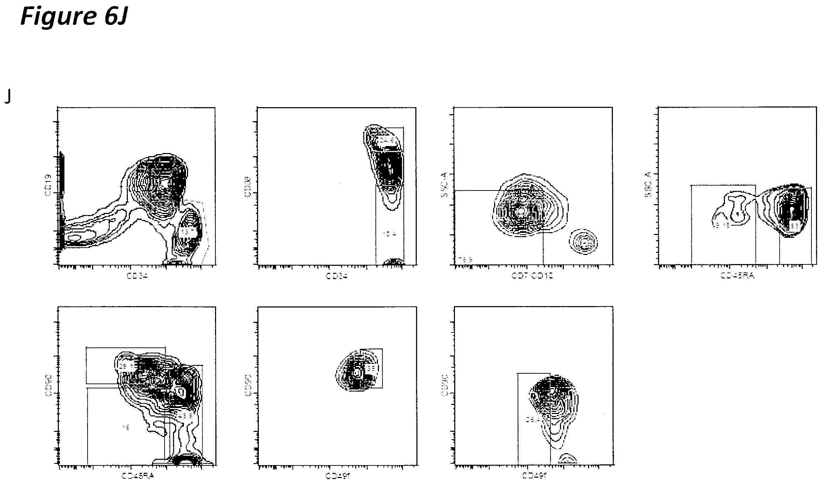

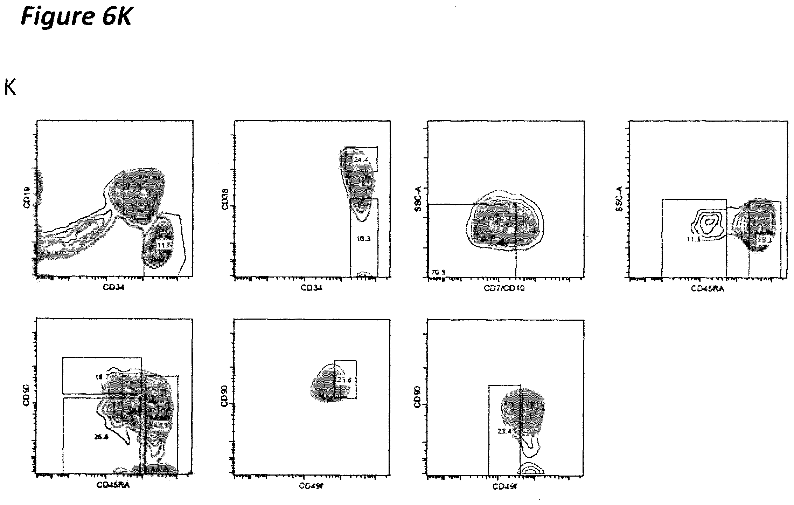

Figure 6 shows (A) Chimerism as measured by flow cytometry for CD45 in non-injected bones of 16 week transplanted mice of control or 4HPR cultured CB cells at 16 weeks post-transplant. All bones for each mouse were pooled following chimerism analysis and frozen for subsequent CD34 hierarchy analysis. Flow cytometry analysis were performed on CD34+ enriched cells using Miltenyi human CD34 enrichment protocol on thawed samples for the human CB hierarchy at the 16 HSC dose from 4 biological experiments, marked with different symbols (n=4 mice per biological experiment). The number of (B) CD34+CD19- cells, (C) CD34+CD38-, (D) CD34+CD38+, (E) HSC, (F) MPP, (G) MLP, and (I) GMP were quantitated from each mouse at 16 weeks post-transplant. Representative HSC hierarchy analysis scheme of CD34 enriched cells isolated from mice 16 weeks post-transplantation engrafted with ex vivo cultured CB cells in the presence of(J) vehicle control (black) or (K) 4HPR (green).

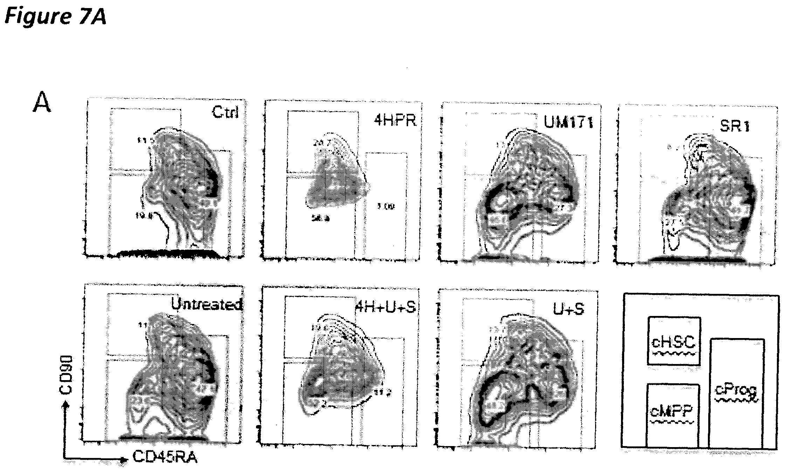

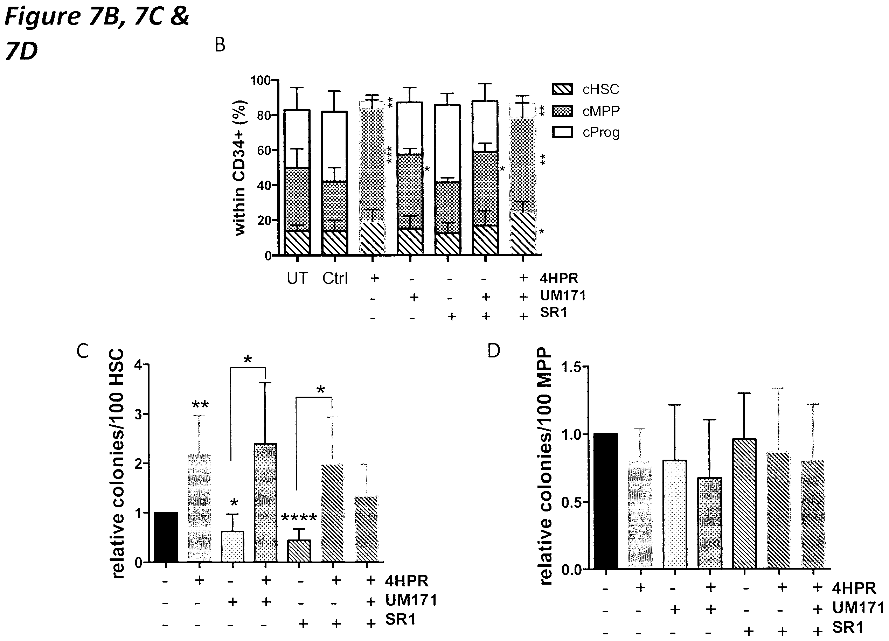

**Figure** 7 shows 4HPR treatment preserves stem cell associated immunophenotypic markers in the primitive population of ex vivo cultured human cord blood. (A) Representative flow cytometry analysis of the primitive hierarchy within the CD34+ fraction of day 8 cultured lin- CB cells after the indicated treatments for CD90 and CD45RA, n=4. B) The distribution of cultured CD34+CD90+CD45RA- (cHSC), CD34+CD90-CD45RA- (cMPP), and CD34+CD90-CD45RA- (cProg) for the indicated conditions at day 8 of ex vivo culture following CD34 enrichment. Statistical significance assayed through unpaired t-test relative to vehicle control (Ctrl). The relative number of colonies resulting from (C) HSC or (D) MPP grown in methylcellulose CFC assays in the presence of vehicle control (-) or the indicated combination of 2 uM 4HPR, or 35nM UM171 or 0.5 uM SR1 at 10 days. Statistical significance is calculated via unpaired t-test relative to ctrl (black) or to the presence of absence of 4HPR treatment for UM171 or SR1.

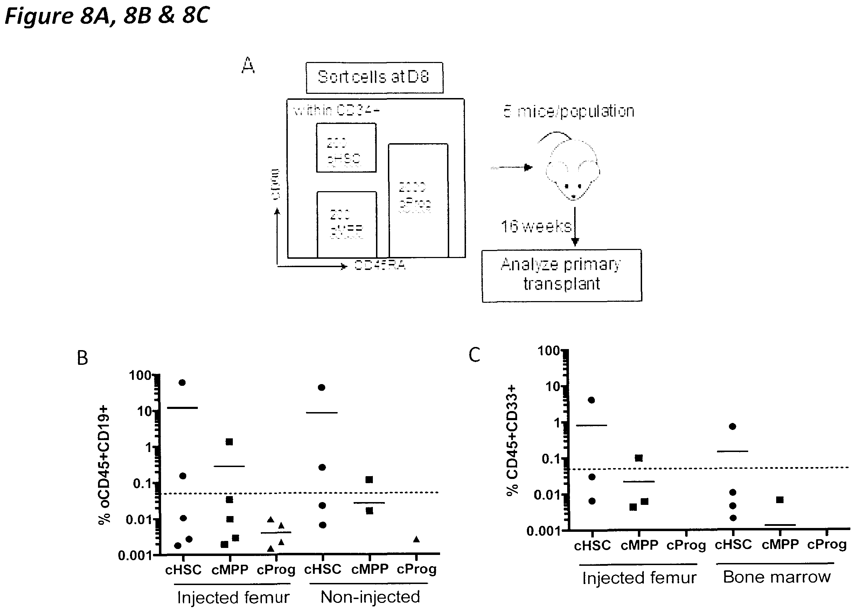

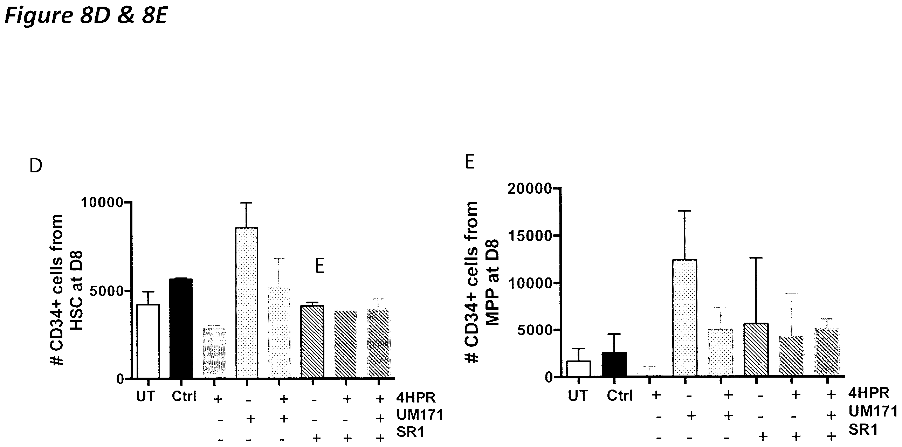

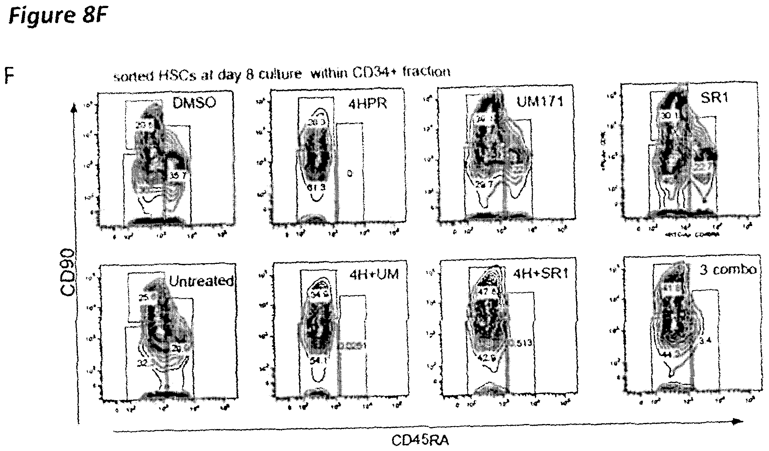

**Figure** 8 shows (A) The indicated cell populations were sorted from the CD34+ fraction of lin-CB cultured for 8 days and transplanted into NSG mice to determine human lymphoid and myeloid engraftment. B) Lymphoid and C) myeloid engraftment from (A) at 16 weeks post-transplantion. The number of CD34+ cells following 8 days culture from 100 sorted D) HSC or E) MPP with the indicated treatments shows that UM171 and SR1 promote CD34+ expansion more from MPP than HSC (n=2). F) Flow cytometry plots showing the CD34+ population from 8 day cultured HSC.

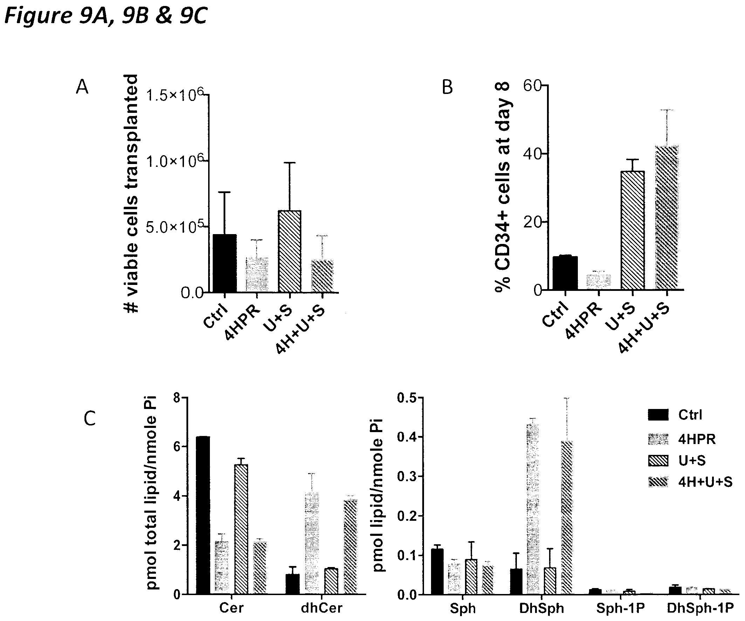

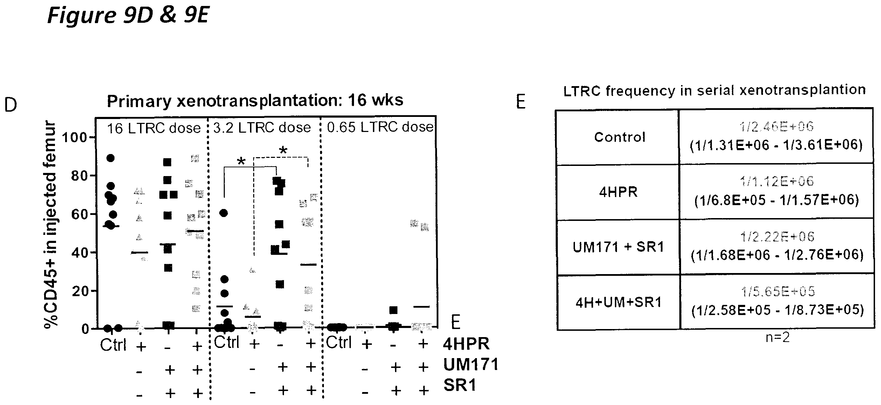

**Figure** 9 shows 4HPR synergizes with UM171 and SR1 to enhance HSC self-renewal in vivo following ex vivo expansion of cord blood. (A) The number of cells following 8 days of ex vivo culture at the 16 LTRC dose grown as in Figure 5A for the indicated drug treatments (n=2). (B) Flow cytometry for CD34+ cells at day 8 culture indicates UM171 and SR1 treatment expands the % CD34+ as previously reported (Fares, et al, 2013). (C) LC/MS analysis for total ceramide (Cer), dihydroceramide (dhCer), sphingosine (Sph), dihydrosphingosine (dhSph), Sph-1P and DhSph-1P levels show that 4HPR increases dhCer and dhSph alone or in combination with UM171 and SR1 (n=2). (D) Human CD45 chimerism in the injected femurs of transplanted mice for the indicated cell doses at 16 weeks xenotransplantion following 8 day ex vivo culture with indicated drugs. UM171 and SR1 ex vivo treatment significantly increases engraftment at the 3.2 LTRC dose. (2 biological replicates) (E) CD45+ cells were isolated from 2 biological experiments of the 16 LTRC dose primary mice and transplanted at limiting doses into secondary recipients for 16 weeks. Human CD45+ marking of >0.01% was considered positive for secondary engraftment. Limiting dilutation analysis was performed using ELDA software and the long-term repopulating cell (LTRC) frequency for each biological replicate plotted and the average LTRC frequency calculated.

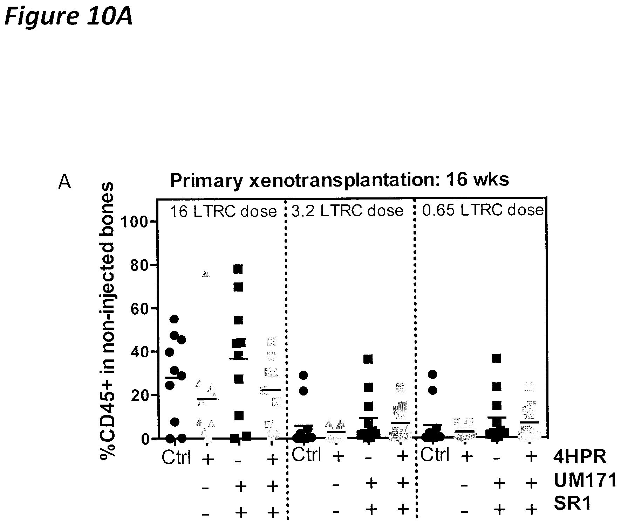

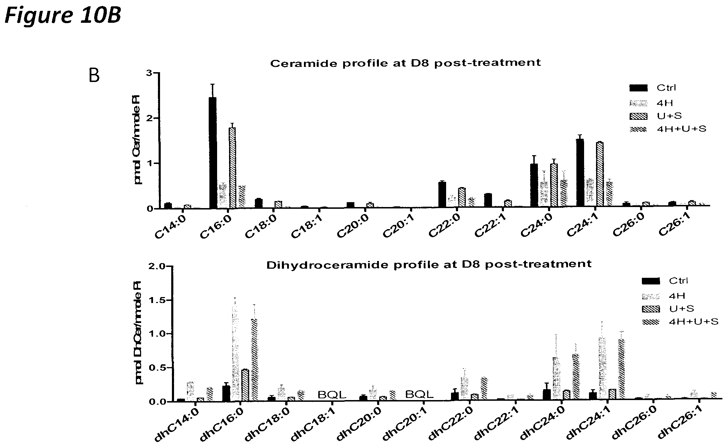

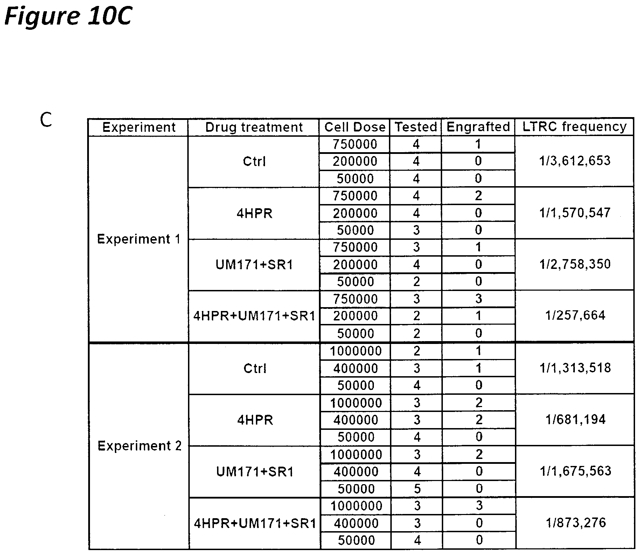

Figure 10 shows (A) CD45 human chimerism in non-injected bones of mice at 16 weeks post-transplantation for the indicated cell doses following 8 day ex vivo culture with control, or combinations of 4HPR, UM171 and SR1. (B) The ceramide and dihydroceramide profiles with the indicated fatty-acyl chain of cells collected following 8 days of culture with indicated treatments measured by LC/MS. (C) The secondary LDA analysis for 2 separate biological experiments showing cell doses transplanted for calculation of LTRC frequency shown in Figure 9D.

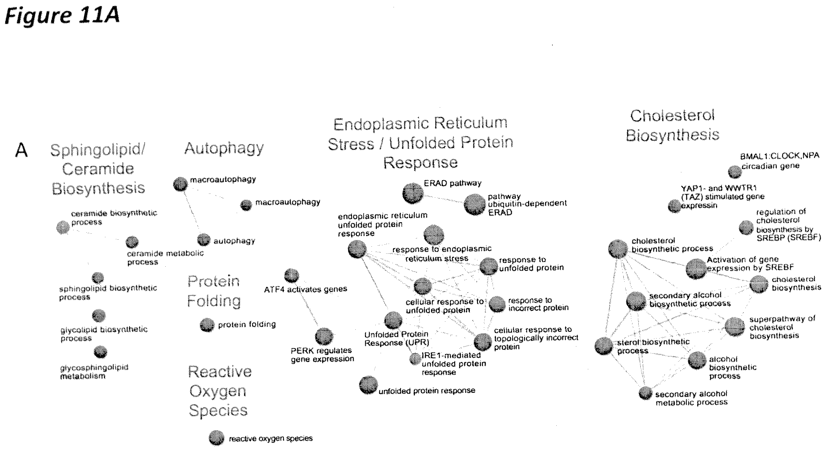

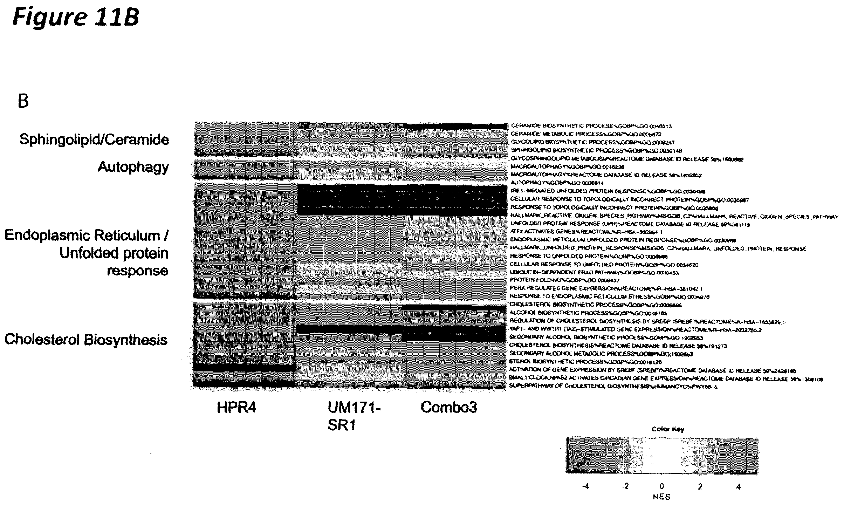

Figure 11 shows 4HPR treatment selectively upregulates sphingolipid biosynthesis and autophagy gene expression in human cord blood. RNA from 3 pools of Lin- CB cells treated with vehicle control, 4HPR, UM171+SR1 or the triple combination of 4HPR+UM171+SR1 for2 days were subjected to RNA-sequencing without amplification on the Illumina HiSeq 2500 . (A) Functional enrichment map of gene sets enriched (red) by 4HPR treatment including or enriched by Ctrl treatment (blue) at day 2. (B) Heatmap of those gene sets from Fig 11A and their expression relative to Ctrl for the treatment groups shows that the combination of 4HPR with UM171+SR1 give an intermediate gene expression where sphingolipid biosynthesis, autophagy and ER/UPR gene sets are still enriched in the Combo3 treatment.

### DETAILED DESCRIPTION

In the following description, numerous specific details are set forth to provide a thorough understanding of the invention. However, it is understood that the invention may be practiced without these specific details.

Worldwide 50,000 HSCT (hematopoietic stem cell transplant) procedures are undertaken annually to treat blood disorders and cancers, yet 2/3 of patients who need HSCT lack matched donor tissue. Improvements in hematopoietic stem cell (HSC) availability, and in efficacy and safety of HSCT procedures would extend applicability to larger numbers of cancer patients and beyond, for example to those who could benefit from gene repair.

Lipid signaling pathways have proven important in regenerative medicine as short-term treatment with prostagladin E2 (PGE2) enhances engraftment of CB and is in clinical trials for efficacy in transplant protocols (North, et al). Autophagy is critical for maintaining lipid homeostasis under nutrient stress in mice (Singh, Nature, 2009). We find that lipid metabolism and signaling genes are differentially expressed in HSC compared to progenitor cells, with those 23 genes high in HSC termed a lipid-stem signature through high resolution transcriptome analysis of the human hematopoietic hierarchy (Manuscript in preparation). Many eicosanoid metabolism genes including those that regulate PGE2 production are in the lipid stem signature. Intriguingly, sphingolipid metabolism, a related lipid pathway which makes the bioactive signaling lipids sphingosine-1-phosphate (S1P) and ceramide-1-phosphate (C1P), is transcriptionally and functionally distinct between HSC and progenitors (Fig 2A) (reviewed in Merrill, et al, 2011). DEGS1 is the final enzyme in de novo sphingolipid synthesis that converts dihydroceramide to ceramide, the central component to all sphingolipids (Slddique, review, JBC, 2015). These key lipid metabolites are synthesized at the ER and then further processed at the golgi and lysosome. Mouse cells deficient for DEGS1 have been described to activate autophagy under nutrient stress (Siddique, MCB, 2013). Fenrentinide/4HPR is a pleitrophic molecule that irreversibly inhibits DEGS1 activity with prolong exposure at micromolar concentrations in vitro (Rahmanlyan, et al, JBC, 2011). Such inhibition has been linked to accumulation of dihydroceramides and activation of ER stress and autophagy in a multitude of cell lines (reviewed in Li, et al, Cell death and Disease, 2014). We asked whether 4HPR treatment could induce the distinctive stress response of the HSC to activate autophagy to specifically protect CB HSC self-renewal function through the metabolic stress of ex vivo expansion for BMT.

Clinically, single cord blood (CB) units do not contain enough short (ST) and long term (LT) HSC to enable HSCT into adult recipients, prompting the development of ex vivo CB expansion methods. Numerous conditions yield ST-HSC/progenitor expansion, but LT-HSC expansion is neither robust nor reliable. LT-HSC have properties distinct from those of ST-HSC and more committed progenitors, including altered cell cycle properties, stress responses and metabolism. Current strategies to expand primitive CD34+ cells in the presence of cytokines disrupt many of these HSC properties, leading to release from quiescence, increased reactive oxygen species (ROS) and activation of stress responses. Autophagy is an essential process for protection of mouse LT-HSC from metabolic stress and aging. We have uncovered a lipid-stem signature, which includes the *de novo* sphingolipid synthesis enzyme dihydroceramide desaturase (DEGS1), that is transcriptionally and functionally distinct between HSC and committed progenitors. Genetic or pharmacological disruption of DEGS1 has been reported to induce autophagy, including with the pleiotropic retinoid fenretinide/*N*-(4-hydroxyphenyl) retinamide (4HPR). Therefore, we asked if 4HPR treatment in ex *vivo* expansion regimens of human CB would improve LT-HSC function *in vivo.* Dihydroceramide levels are dramatically increased in CB cells cultured with 4HPR. 4HPR increases LC3 cleavage and autophagy as detected by CYTO-ID, selectively in primitive CD34+CD38-but not more committed CD34+CD38+ cells, under cytokine-rich culture conditions or upon short-term cytokine withdrawal. Treated cells exhibit decreased ROS and mitochondrial membrane potential, reflective of the metabolic state of quiescent HSC. In ex vivo culture, 4HPR treatment for 8d alone or in combination with known CB ex vivo expansion agents SR1 or UM171 remarkably limits the expansion of CD34+CD45RA+ committed progenitors while preserving immunophenotypic LT- and ST-HSC. 4HPR dramatically induces enrichment of sphingolipid/ceramide biology, unfolded protein response, and macroautophagy gene sets in CB cells alone or in combination with UM171 and SR. Importantly, in limiting dilution serial transplantation assays, the frequency of LT-HSC was increased by ex vivo 4HPR treatment compared to controls, and doubled when 4HPR was added to SR1+UM171 treatment compared to SR1+UM171 treatment alone. Here, we describe a previously unrecognized function of 4HPR in preserving human LT-HSC function while still allowing ex vivo expansion of ST-HSC and progenitors. We propose that autophagy activation in primitive CB cells with 4HPR as a novel strategy to improve clinical HSCT outcome.

In an aspect not covered by the appended claims, there is provided a method for improving or preserving hematopoietic stem cell (HSC) self-renewal during *ex-vivo* expansion of a population of cells comprising HSCs in an expansion medium, comprising culturing the population of cells in the presence of N-(4hydroxyphenyl)retinamide (4HPR).

As used herein *"hematopoietic stem cell"* refers to a cell of bone marrow, liver, spleen or cord blood in origin, capable of developing into any mature myeloid and/or lymphoid cell. Stems cells have the ability to *"self-renew",* that is the ability to go through numerous cycles of cell division while maintaining the undifferentiated state.

As used herein *"medium"* refers to a growth medium or culture medium designed to support the growth hematopoietic stem cells. Such medium are known in the art, including mediums used for expanding a population of cells, and in particular hematopoietic stem cells, and may be referred to as an *"expansion medium".*

In some aspects, the population of cells are derived from cord blood.

In some aspects, the population is CD34+ enriched.

In some aspects, the population of HSCs are CD34+/CD133+.

In some aspects not covered by the appended claims, the method further comprises culturing the population of cells in the presence of StemRegenin-1 (SR1), (1r,4r)-N1-(2-benzyl-7-(2-methyl-2H-tetrazol-5-yl)-9H-pyrimido[4,5-b]indol-4-yl)cyclohexane-1,4-diamine (UM171) or both.

In some aspects, hematopoietic stem cell (HSC) self-renewal is improved or preserved by enriching for HSCs.

In an aspect not covered by the appended claims, there is provided a method for enriching for hematopoietic stem cell (HSC) during ex-vivo expansion of a population of cells comprising HSCs in an expansion medium, the method comprising culturing the population of cells in the presence of N-(4hydroxyphenyl)retinamide (4HPR).

In an aspect, there is provided a use of N-(4hydroxyphenyl)retinamide (4HPR) for improving or preserving hematopoietic stem cell (HSC) renewability during *ex-vivo* expansion of a population of cells comprising HSCs in an expansion medium.

In an aspect not covered by the appended claims, there is provided N-(4hydroxyphenyl)retinamide (4HPR) for use in improving or preserving hematopoietic stem cell (HSC) renewability during *ex-vivo* expansion of a population of cells comprising HSCs in an expansion medium.

In an aspect not covered by the appended claims, there is provided a medium for culturing/expanding hematopoietic stem cells, comprising N-(4hydroxyphenyl)retinamide (4HPR).

In some aspects, the medium further comprises StemRegenin-1 (SR1), (1r,4r)-N1-(2-benzyl-7-(2-methyl-2H-tetrazol-5-yl)-9H-pyrimido[4,5-b]indol-4-yl)cyclohexane-1,4-diamine (UM171) or both.

In an aspect not covered by the appended claims, there is provided a population of cells expanded using the methods described herein.

The advantages of the present invention are further illustrated by the following examples. The examples and their particular details set forth herein are presented for illustration only and should not be construed as a limitation on the claims of the present invention.

### EXAMPLES

The *de novo* sphingolipid synthesis enzyme DEGS1 is transcriptionally higher in HSC than committed progenitors (Fig 2B) and its' activity can be irreversibly inhibited by 4HPR, a pleiotrophic retinoid agonist explored as a chemotherapeutic agent in several clinical trials with growth inhibitory functions (Fig 2A, B). However, the functional consequence of 4HPR treatment on human cord blood primitive cells is unknown. A dose titration of 4HPR was performed on lineage-depleted CB (lin-CB) cells and 2 uM was calculated to give half maximal cell viability at 3 days post-treatment compared to vehicle control (ctrl), a concentration reported in cell lines to antagonize DEGS1 enzymatic function, and used for all subsequent experiments unless otherwise indicated (Fig 1A). As expected, 4HPR treatment decreased proliferation rate in HSPC subpopulations including HSC, MPP (multipotent progenitor or ST-HSC) and GMP (granulocyte macrophage progenitor) as assayed by BrdU incorporation at day 3 post-treatment (Fig 1B). However, these populations can still transit through the cell cycle in the presence of 4HPR as cell cycle analysis at day 2 or day 3 with Ki67 and Hoescht shows no significant difference between ctrl and 4HPR (Fig 2C, D). Lipid mass spectrometry was performed on lin-CB cells cultured for 8 days to confirm that 4HPR treatment does in fact inhibit DEGS1 activity and increase total dihydroceramide and decrease total ceramide compare to control (Fig 1C and Fig 10C). The levels of the signaling lipid S1P are not altered, which again suggests that while this concentration of 4HPR has growth attenuating effects, lin-CB cells are still able to proliferate with extended treatment. Next, we utlized the colony forming assay (CFC) for in vitro progenitor function to determine if 4HPR treatment alters functional output from isolated populations of CB HSPC. Remarkably, we find that 4HPR treatment specifically of HSC, but not MPP or GMP significantly increases colony output 50% over control treatment (Fig 1D). Resultant colonies are more likely to be granulocyte-macrophage (GM) colonies in both HSC and MPP samples treated with 4HPR (Fig 1E). Flow cytometry for myeloid (CD14) and erythroid (GlyA) markers of all cells from the CFC assays shows that 4HPR enhances myeloid output at the expense of erythroid differentiation (Fig 1F). This enhancement of colony output is independent of retinoid signaling as all-trans retinoic acid (ATRA) treatment in such assays decreases colony output from all HSPC populations (Fig 1G). This is unsurprising as retinoic acid signaling is a potent differentiation signal in hematopoietic cells. Thus, 4HPR inhibition of sphingolipid homeostasis selectively enhances cloning efficiency in human HSC.

We hypothesize that such selective enhancement of CFC cloning efficiency with 4HPR treatment is a result of activating autophagy in HSC in human CB. Warr et all had previously shown that mouse HSC, but not comitted progenitors activate autophagy under stress stimuli. Therefore, we chose to compare the action of 4HPR in autophagy induction on CD34+CD38- (stem)-enriched cells and CD34+CD38+ progenitor-enriched (prog) cells at day 2 post-treatment relative to vehicle control with two independent methods: 1) immunofluorescence (IF) for the autophagosome marker LC3II by confocal microscopy with and without Baflomycin A1 (BAF) to inhibit autophagosome turnover (Fig 3A-C, Fig 4A) and 2) flow cytometry with cyto-ID that stains autophagosomes (Fig 3D-F, Fig 4E-G). LC3II IF microscopy shows that both ctrl and 4HPR-treated stem and progenitor cells at day 2 have basal autophagy (Fig 3A, B). However, in the presence of BAF, only 4HPR-treated stem cells, but not prog cells exhibit an upregulation of autophagy with an increase in LC3II foci area relative to ctrl (Fig 3B, C). Similarly, flow cytometry with cyto-ID staining with vehicle (0 uM) compared to 0.2 uM or 2 uM 4HPR showed autophagic flux is only significantly induced in stem but not prog cells with 4HPR (Fig 3D, E). A decrease in cyto-ID MFI represents more autophagic flux. As reported for mouse HSPC, cytokine withdrawal in human CB stem activates autophagy as measured by cytolD, but not in human prog cells (Fig 3F, 4F, 4G), which is further enhanced in the presence of 4HPR. Autophagy induction with inhibition of mTORC1 by rapamycin decreases cloning efficiency in CFC assays from HSC, MPP and GMP which suggests that 4HPR action is independent of mTORC1 signaling (Fig 4B, C). Rapamycin treatment in ex vivo culture of CB has been reported to enhance engraftment in xenotransplantation which we suspect is due to enhancement of MPP function as suggested by secondary CFC assays (Fig 4C). Autophagy regulates cellular metabolism in mouse HSC (Ho, et al, Nature 2017) to maintain lower metabolic activity particularly in the mitochondria. Therefore, we used flow cytometry to analyzed three metabolic parameters with 4HPR treatment in CB stem and prog-cells at day 2 and day 3 of treatment (Fig 3G-I, Fig 4I-K), and found that 4HPR resulted in decreased ROS and mitochondrial membrane potential and decreased mitochondrial mass potentially through mitophagy in both stem and prog cells. HSC have lower mitochondrial content than progenitors (Fig 4H). Thus, 4HPR induces autophagy specifically in CB stem-enriched cells resulting in a less metabolically active state, reminiscent of more quiescent stem cells

To determine if 4HPR preserves functional HSC in CB liquid culture, we devised an experimental protocol to test the effect of 4HPR treatment in short-term ex vivo expansion culture on repopulation in serial NSG xenotransplantation to functionally read out LT-HSC (Fig 5A). Briefly, lin- CB (enriched for CD34+) is cultured ex vivo in an in vitro limiting dilution assay (LDA) fashion based on 3 initial cell doses with high (16 functional HSC or LTRC), medium (3.2 LTRC) and limiting (0.65 LTRC) in xenotransplantation. All expanded cells are unbiasly transplanted via intrafemoral injection into NSG mice after 8 days of culture where drug is added every 2 day and reconstitution is assayed 16 weeks following transplantation. Flow cytometry analysis over the 8 days culture shows 4HPR treated cultures have fewer immunophenotypic CD34+ cells, enhanced myeloid differentiation with decreased erythroid differentiation(Fig 5B-F). Xenotransplantation data shows that 4HPR expanded CB cells exhibit the same CD45 chimerism as vehicle control through all cell doses even though they have fewer CD34 immunophenotypic cells following ex vivo expansion (Fig 5G, Fig 6A). Similarly, lineage distribution for B lymphoid, myeloid, and erythroid at the 16 HSC dose is comparable between ctrl and 4HPR transplanted cells (Fig 5H-J). Most importantly, primitive lineage negative CD34+ cells are equally prevalent in the mice transplanted with the progeny of ctrl or 4HPR treated 16 LTRC dose cells (Fig 5K). Analysis of the HSC hierarchy in individual mice confirm that all analyzed HSPC populations by flow cytometry shows are on average identical, with similar experimental variation between mice transplanted with control and 4HPR expanded cells (Fig 6C-K, 7 separate experiments). Human CD45+ cells are isolated from primary mice transplanted with the 16 LTRC dose and serially transplanted in LDA fashion to enumerate functional HSC in the gold-standard for long-term repopulating cell (LTRC). Remarkably, 4HPR treated cells show a 3 fold increase in SRC over control treatment through 32 weeks of xenotransplantation with an average LTRC frequency of 1e6 CD45+ cells (Fig 5L, n=5, p=0.02). Fresh CD34+ CB through 32-40 weeks of xenotransplantion typically gives LTRC frequency between 1/5e5 to 1/8e5 using similar serial transplantation methods suggesting ex vivo expansion culture decreases LTRC frequency that is partially restored by 4HPR. Thus, 4HPR acts to preserve LT-HSC function in ex vivo expansion despite limiting total CD34+ cells during culture.

Given the CD34 compartment where functional HSC should reside following ex vivo expansion is critical to blood production, we asked if there were immunophenotypic alterations following 4HPR treatment. StemRegenin-1 (SR1) or UM171 are two molecules whose action on CB has been reported to expand CD34+ cells dramatically and lead to increase reconstitution in xenotransplantation (Fares, et al Science, 2014; Boitano, Science, 2010). We sought to ask if limiting CD34+ expansion with 4HPR treatment is synergistic with these known agonists of CB ex vivo expansion. Lin- CB were cultured with these 3 small molecules alone or in combination for 8 days and the CD34 compartment was isolated and analyzed by flow cytometry further with CD90 and CD45RA to look at immunophenotypic cultured HSC (cHSC) , MPP (cMPP) and committed progenitors (cProg). These populations were sorted from lin-CB at D8 following expansion and transplanted into NSG mice for 16 weeks to ascertain that cProg cells are committed progenitors, while cHSC and cMPP retain multipotency following culture (Fig 8A, B). Remarkably, 4HPR significantly limits the percentage of (cProg) cells while increasing cMPP in the CD34 compartment and trends to increasing cHSC relative to control. To further elucidate the specific action of 4HPR on HSC over MPP and compare this to UM171 and SR1, sorted HSC and MPP were put into ex vivo expansion conditions for 8 days with the same treatments as in Fig 7A, B and their growth and CD34+ compartment were monitored by flow cytometry (Fig 8D-F). Consistent with the literature, UM171 dramatically increases primitive CD34+ from both HSC and MPP at D8 after ex vivo expansion. SR1 expansion of CD34+ subpopulations occurs primarily in sorted MPP, not HSC. Remarkably, 4HPR treatment limits the formation of cProg cells in lin- CB as well as HSC and MPP and is dominant over UM171 or SR1 singularly and in combination (Fig 7A, 7B, 8D, 8F). To determine whether the activity of 4HPR is dominant or synergistic with UM171/SR1 in cloning efficiency, we used the CFC assay on sorted HSC and MPP treated with the indicated small molecules (Fig 7C,D). Neither UM171 nor SR1 altered relative number of colonies relative to the vehicle control, but both decreased cloning efficiency of the HSC (Fig 5B, C). However, 4HPR (green) increased relative cloning efficiency from HSC in combination with either UM171 or SR1 to levels similar to 4HPR alone (Fig 7C). Thus, we hypothesize that the combination of 4HPR with UM171 and SR1 may together enhance HSC self-renewal while allowing for expansion of LT- and ST-HSC from CB in culture.

To determine if there is synergism for HSC self-renewal following CB ex vivo expansion between 4HPR and UM171/SR1, 2 pools of lin- CB cells were treated with vehicle control, 4HPR, UM171+SR1 or 4HPR+UM171+SR1 (Combo3 ) through 8 day ex vivo expansion, transplanted into NSG mice and then serial transplanted with LDA to enumerate LTRC frequency as in Fig 5A (Fig 9).

Combo3 treated cells shared characteristics of both UM171/SR1 treatment as well as 4HPR treatment. While 4HPR treatment restricted the total expansion of cells at d8 for Combo3 (Fig 9A), the CD34% percentage within expanded cells was greatly increased to levels comparable to UM171/SR1 treatment (Fig 9B). The sphingolipid profile of Combo3 D8 expanded cells is identical to 4HPR expanded cells (Fig 9C, Fig 10B). As expected, UM171 and SR1 treatment UM171/SR1 or Combo3 treatment qualitatively improves average primary engraftment of ex vivo CB cells with lower doses of HSC (Fig 9D) relative to vehicle control consistent with UM171/SR1's ability to expand CD34+ cells ex vivo (Fig 9B). Although the LTRC frequency of UM171/SR1 treated cells in serial xenotransplantation is not improved over vehicle control (Fig 7E) in our hands, the 3 combo drug treatment of 4HPR/SR1/UM171 results in 4 fold LTRC frequency over control. The resultant LTRC frequency is consistent with the frequency we see from mice transplanted with uncultured CB suggesting this drug combination may be clinically relevant for ex vivo expansion of CB for HSCT to preserve HSC function.

To elucidate the genetic pathways enriched by 4HPR treatment in human CB that may contribute to preserving HSC function in ex vivo expansion alone or in combination with UM171 and SR1, we performed RNA-sequencing of 3 pools of lin-CB at day 2 and day 4 following treatment with all four treatment groups. To avoid amplification bias, RNA-seq was performed on samples without amplification. Consistent with the phenotypic analyses described in Fig 3, 4HPR treatment enriched for autophagy gene sets including mitophagy (Fig 11A). As suggested in the literature, 4HPR treatment upregulates ER stress and UPR gene sets in human CB (Fig 11A). Importantly, 4HPR treatment in CB is targeting DEGS1 as lipid metabolism is altered with an enrichment of ceramide/sphingolipid biosynthesis gene sets probably in a attempt to restore ceramide levels due to DEGS1 inhibition and a decrease in cholesterol/sterol biosythetic pathways (Fig 11A). The latter is known to be regulated by autophagy (Singh, Nature, 2009).

4HPR acts dominantly to limit CD34+CD45RA+ progenitor expansion in CB ex vivo cultures in combination with SR1 and UM171. This 3 combo drug treatment of 4HPR/SR1/UM171 following ex vivo culture results in a synergistic increase in HSC self-renewal proven by secondary xenotransplantion. Thus, we propose that specifically targeting pathways important for HSC can be efficiacious for identifying promising agents such as 4HPR in the preservation of HSC self-renewal during ex vivo expansion of CB.

### Reference List

1. Benveniste P, Cantin C, Hyam D, Iscove NN. Hematopoietic stem cells engraft in mice with absolute efficiency. Nat Immunol 2003;4: 708-13.
2. Lai W-L, Wong N-S. The PERK/eIF2α signaling pathway of Unfolded Protein Response is essential for N-(4-hydroxyphenyl)retinamide (4HPR)-induced cytotoxicity in cancer cells. Experimental Cell Research 2008;314: 1667-82.
3. Mathew R, Karp CM, Beaudoin B, Vuong N, Chen G, Chen HY, Bray K, Reddy A, Bhanot G, Gelinas C, Dipaola RS, Karantza-Wadsworth V, et al. Autophagy suppresses tumorigenesis through elimination of p62. Cell 2009;137: 1062-75.
4. Singh R, Kaushik S, Wang Y, Xiang Y, Novak I, Komatsu M, Tanaka K, Cuervo AM, Czaja MJ. Autophagy regulates lipid metabolism. Nature 2009;458: 1131-5.
5. Benveniste P, Frelin C, Janmohamed S, Barbara M, Herrington R, Hyam D, Iscove NN. Intermediate-term hematopoietic stem cells with extended but time-limited reconstitution potential. Cell Stem Cell 2010;6: 48-58.
6. Boitano AE, Wang J, Romeo R, Bouchez LC, Parker AE, Sutton SE, Walker JR, Flaveny CA, Perdew GH, Denison MS, Schultz PG, Cooke MP. Aryl hydrocarbon receptor antagonists promote the expansion of human hematopoietic stem cells. Science 2010;329: 1345-8.
7. Liu XW, Su Y, Zhu H, Cao J, Ding WJ, Zhao YC, He QJ, Yang B. HIF-1alpha-dependent autophagy protects HeLa cells from fenretinide (4-HPR)-induced apoptosis in hypoxia. Pharmacol Res 2010;62: 416-25.
8. Eppert K, Takenaka K, Lechman ER, Waldron L, Nilsson B, van Galen P, Metzeler KH, Poeppl A, Ling V, Beyene J, Canty AJ, Danska JS, et al. Stem cell gene expression programs influence clinical outcome in human leukemia. Nat Med 2011;17: 1086-93.
9. Goessling W, Allen RS, Guan X, Jin P, Uchida N, Dovey M, Harris JM, Metzger ME, Bonifacino AC, Stroncek D, Stegner J, Armant M, et al. Prostaglandin E2 enhances human cord blood stem cell xenotransplants and shows long-term safety in preclinical nonhuman primate transplant models. Cell Stem Cell 2011;8: 445-58.
10. Merrill AH, Jr. Sphingolipid and glycosphingolipid metabolic pathways in the era of sphingolipidomics. Chem Rev 2011;111: 6387-422.
11. Notta F, Doulatov S, Laurenti E, Poeppl A, Jurisica I, Dick JE. Isolation of single human hematopoietic stem cells capable of long-term multilineage engraftment. Science 2011;333: 218-21.
12. Ouimet M, Franklin V, Mak E, Liao X, Tabas I, Marcel YL. Autophagy regulates cholesterol efflux from macrophage foam cells via lysosomal acid lipase. Cell Metab 2011;13: 655-67.
13. Rahmaniyan M, Curley RW, Obeid LM, Hannun YA, Kraveka JM. Identification of Dihydroceramide Desaturase as a Direct in Vitro Target for Fenretinide. The Journal of Biological Chemistry 2011;286: 24754-64.
14. Csaszar E, Kirouac DC, Yu M, Wang W, Qiao W, Cooke MP, Boitano AE, Ito C, Zandstra PW. Rapid expansion of human hematopoietic stem cells by automated control of inhibitory feedback signaling. Cell Stem Cell 2012;10: 218-29.
15. Doulatov S, Notta F, Laurenti E, Dick JE. Hematopoiesis: a human perspective. Cell Stem Cell 2012;10: 120-36.
16. Lechman ER, Gentner B, van Galen P, Giustacchini A, Saini M, Boccalatte FE, Hiramatsu H, Restuccia U, Bachi A, Voisin V, Bader GD, Dick JE, et al. Attenuation of miR-126 activity expands HSC in vivo without exhaustion. Cell Stem Cell 2012;11: 799-811.
17. Taniguchi M, Kitatani K, Kondo T, Hashimoto-Nishimura M, Asano S, Hayashi A, Mitsutake S, Igarashi Y, Umehara H, Takeya H, Kigawa J, Okazaki T. Regulation of autophagy and its associated cell death by "sphingolipid rheostat": reciprocal role of ceramide and sphingosine 1-phosphate in the mammalian target of rapamycin pathway. J Biol Chem 2012;287: 39898-910.
18. Chanda B, Ditadi A, Iscove NN, Keller G. Retinoic acid signaling is essential for embryonic hematopoietic stem cell development. Cell 2013;155: 215-27.
19. Laurenti E, Doulatov S, Zandi S, Plumb I, Chen J, April C, Fan J-B, Dick JE. The transcriptional architecture of early human hematopoiesis identifies multilevel control of lymphoid commitment. Nature Immunology 2013;14: 756-63.
20. Siddique MM, Li Y, Wang L, Ching J, Mal M, Ilkayeva O, Wu YJ, Bay BH, Summers SA. Ablation of dihydroceramide desaturase 1, a therapeutic target for the treatment of metabolic diseases, simultaneously stimulates anabolic and catabolic signaling. Mol Cell Biol 2013;33: 2353-69.
21. Warr MR, Binnewies M, Flach J, Reynaud D, Garg T, Malhotra R, Debnath J, Passegué E. FOXO3A directs a protective autophagy program in haematopoietic stem cells. Nature 2013.
22. Fares I, Chagraoui J, Gareau Y, Gingras S, Ruel R, Mayotte N, Csaszar E, Knapp DJ, Miller P, Ngom M, Imren S, Roy DC, et al. Cord blood expansion. Pyrimidoindole derivatives are agonists of human hematopoietic stem cell self-renewal. Science 2014;345: 1509-12.
23. Li Y, Li S, Qin X, Hou W, Dong H, Yao L, Xiong L. The pleiotropic roles of sphingolipid signaling in autophagy. Cell Death Dis 2014;5: e1245.
24. Luo Y, Li L, Zou P, Wang J, Shao L, Zhou D, Liu L. Rapamycin enhances long-term hematopoietic reconstitution of ex vivo expanded mouse hematopoietic stem cells by inhibiting senescence. Transplantation 2014;97: 20-9.
25. Maceyka M, Spiegel S. Sphingolipid metabolites in inflammatory disease. Nature 2014;510: 58-67.
26. van Galen P, Kreso A, Mbong N, Kent DG, Fitzmaurice T, Chambers JE, Xie S, Laurenti E, Hermans K, Eppert K, Marciniak SJ, Goodall JC, et al. The unfolded protein response governs integrity of the haematopoietic stem-cell pool during stress. Nature 2014;510: 268-72.
27. van Galen P, Kreso A, Wienholds E, Laurenti E, Eppert K, Lechman ER, Mbong N, Hermans K, Dobson S, April C, Fan JB, Dick JE. Reduced lymphoid lineage priming promotes human hematopoietic stem cell expansion. Cell Stem Cell 2014;14: 94-106.
28. Koberlin MS, Snijder B, Heinz LX, Baumann CL, Fauster A, Vladimer GI, Gavin AC, Superti-Furga G. A Conserved Circular Network of Coregulated Lipids Modulates Innate Immune Responses. Cell 2015;162: 170-83.
29. Laurenti E, Frelin C, Xie S, Ferrari R, Dunant CF, Zandi S, Neumann A, Plumb I, Doulatov S, Chen J, April C, Fan JB, et al. CDK6 levels regulate quiescence exit in human hematopoietic stem cells. Cell Stem Cell 2015;16: 302-13.
30. Lund TC, Boitano AE, Delaney CS, Shpall EJ, Wagner JE. Advances in umbilical cord blood manipulation [mdash] from niche to bedside. Nature Reviews Clinical Oncology 2015;12: 163-74.
31. Delaney C. Expansion of Human Hematopoietic Stem Cells. Thomas' Hematopoietic Cell Transplantation, Fifth Edition 2016: 62-70.
32. Garcia-Prat L, Martinez-Vicente M, Perdiguero E, Ortet L, Rodriguez-Ubreva J, Rebollo E, Ruiz-Bonilla V, Gutarra S, Ballestar E, Serrano AL, Sandri M, Munoz-Canoves P. Autophagy maintains stemness by preventing senescence. Nature 2016;529: 37-42.
33. Lechman ER, Gentner B, Ng SW, Schoof EM, van Galen P, Kennedy JA, Nucera S, Ciceri F, Kaufmann KB, Takayama N, Dobson SM, Trotman-Grant A, et al. miR-126 Regulates Distinct Self-Renewal Outcomes in Normal and Malignant Hematopoietic Stem Cells. Cancer Cell 2016;29: 214-28.
34. Notta F, Zandi S, Takayama N, Dobson S, Gan OI, Wilson G, Kaufmann KB, McLeod J, Laurenti E, Dunant CF, McPherson JD, Stein LD, et al. Distinct routes of lineage development reshape the human blood hierarchy across ontogeny. Science 2016;351: aab2116.
35. Wagner JE, Brunstein CG, Boitano AE, DeFor TE, McKenna D, Sumstad D, Blazar BR, Tolar J, Le C, Jones J. Phase I/II Trial of StemRegenin-1 Expanded Umbilical Cord Blood Hematopoietic Stem Cells Supports Testing as a Stand-Alone Graft. Cell Stem Cell 2016;18: 144-55.
36. Ho TT, Warr MR, Adelman ER, Lansinger OM, Flach J, Verovskaya EV, Figueroa ME, Passegue E. Autophagy maintains the metabolism and function of young and old stem cells. Nature 2017;543: 205-10.

## Claims

1. Use of N-(4hydroxyphenyl)retinamide (4HPR) for improving or preserving hematopoietic stem cell (HSC) renewability during *ex-vivo* expansion of a population of cells comprising HSCs in an expansion medium.

2. The use of claim 1, wherein the population of cells are derived from cord blood.

3. The use of claim 1, wherein the population is CD34+ enriched.

4. The use of claim 1, wherein the population of HSCs are CD34+/CD133+.

5. The use of claim 1, said use further comprising use of StemRegenin-1 (SR1), (1r,4r)-N1-(2-benzyl-7-(2-methyl-2H-tetrazol-5-yl)-9H-pyrimido[4,5-b]indol-4-yl)cyclohexane-1,4-diamine (UM171) or both.

6. The use of any one of claims 1-5, wherein hematopoietic stem cell (HSC) self-renewal is improved or preserved by enriching for HSCs.

## Patentansprüche

1. Verwendung von N-4Hydroxyphenyl)retinamid (4HPR) zur Verbesserung oder Bewahrung der Erneuerbarkeit hämatopoetischer Stammzellen (HSC) bei der Ex-vivo-Expansion einer HSC umfassenden Population von Zellen in einem Expansionsmedium.

2. Verwendung nach Anspruch 1, wobei die Population von Zellen aus Nabelschnurblut stammt.

3. Verwendung nach Anspruch 1, wobei die Population CD34+-angereichert ist.

4. Verwendung nach Anspruch 1, wobei die Population von HSC CD34+/CD133+ ist.

5. Verwendung nach Anspruch 1, wobei die Verwendung ferner Verwendung von StemRegenin-1 (SR1) oder bzw. und (1r,4r)-N1-(2-Benzyl-7-(2-methyl-2H-tetrazol-5-yl)-9H-pyrimido[4,5-b]indol-4-yl)cyclohexan-1,4-diamin (UM171) umfasst.

6. Verwendung nach einem der Ansprüche 1-5, wobei Selbsterneuerung hämatopoetischer Stammzellen (HSC) durch Anreichern auf HSC verbessert oder bewahrt wird.

## Revendications

1. Utilisation de N-(4-hydroxyphényl)rétinamide (4HPR) pour l'amélioration ou la préservation de la renouvelabilité de cellules souches hématopoïétiques (HSC) pendant une expansion ex *vivo* d'une population de cellules comprenant des HSC dans un milieu d'expansion.

2. Utilisation selon la revendication 1, la population de cellules étant issue de sang de cordon.

3. Utilisation selon la revendication 1, la population étant enrichie en CD34+.

4. Utilisation selon la revendication 1, la population de HSC étant CD34+/CD133+.

5. Utilisation selon la revendication 1, ladite utilisation comprenant en outre l'utilisation de StemRegenin-1 (SR1), de (1r,4r)-N1-(2-benzyl-7-(2-méthyl-2H-tétrazol-5-yl)-9H-pyrimido[4,5-b]indol-4-yl)cyclohexane-1,4-diamine (UM171) ou des deux.

6. Utilisation selon l'une quelconque des revendications 1 à 5, l'auto-renouvellement de cellules souches hématopoïétiques (HSC) étant amélioré ou préservé en enrichissant pour des HSC.
